# EUROPEAN PATENT APPLICATION

(11) **EP 3 081 261 A1**
(43) Date of publication of application: **19.10.2016**
(21) Application number: 16000293.7
(22) Date of filing: 05.02.2016
(51) Int. Cl.: A61N 5/06, A61B 18/14

(54) **INTRA-ORAL ELEMENT FOR APPLICATION OF ENERGY DELIVERY TREATMENTS IN JAWBONE, GUMS OR OTHER TISSUES INSIDE THE MOUTH**

(30) Priority: 13.04.2015 ES 201530490
(71) Applicant: Sanchez Jaime, Maria del Pilar, 08970 Barcelona (ES)
(72) Inventor: Sanchez Jaime, Maria del Pilar, 08970 Barcelona (ES)
(74) Representative: Diaz Nunez, Joaquin

(57) **Abstract**

Intra-oral element for application of energy delivery treatments in jawbone, gums or other tissues inside the mouth, comprising a main body (2) attached to a cable (3) that connects it to the energy equipment and to a shaft (4) with a head (5) at its distal end where it incorporates the energy application means (6), formed by electrodes, electric resistances, or a combination of both. It also incorporates a temperature sensor (7), means for quantitative measurement of the energy delivered, and a screen (8) displaying the temperature and/or power, control buttons (9) and emergency stop button (10). The main body (2) is a handle-shaped housing. The shaft (4) is removable and the head (5) is provided with an adapter (51), formed by a conductive medium with flexible structure.

## Description

### OBJECT OF THE INVENTION

The invention, as stated the wording of the present specification, relates to an intra-oral element for application of energy delivery treatments in gums or other tissues inside the mouth.

More particularly, the object of the invention is focused on an accessory device the purpose of which is to be used with an energy equipment, preferably based on currents or electromagnetic pulses or radio frequency waves, but not limited to this, whose design and structural configuration are specifically designed for ergonomic intra-oral use in order to apply physiotherapy treatments, with thermal or athermal effect more or less deep as appropriate, intended to improve blood circulation and/or state of cellular tissue of jawbones, gums or other tissues inside the mouth through its molecular stimulation, serving as an instrument in the treatment or alleviation of oral conditions and/or aid auxiliary element to encourage, for example, the osseointegration of implants.

### FIELD OF APPLICATION OF THE INVENTION

The field of application of the present invention falls within the sector of industry dedicated to the manufacture of equipment, appliances and devices for medical, dental or physiotherapy treatments, focusing particularly on those intended to intra-oral conditions.

### BACKGROUND OF THE INVENTION

As is known, there is a wide variety of means for the therapeutic application of energy, usually in the form of heat, which may be merely superficial or deep. The most used are based on the depth of thermal action and the main mechanism for energy transfer, which may be individually thermal, that is, it produces a detectable heat effect or feeling in the area treated, or "athermal", in which the effect on the treated area is not perceived as heat.

Thus, the superficial means only produce a warming of the body surface, since its penetration is very low, because these are absorbed almost in its entirety cutaneously. For example, the infrared radiation, although it occurs passage of heat to deeper tissues (by conduction or by the convective action of circulation), its therapeutic actions are more by reflex mechanisms than by direct heating of the area.

By contrast, the deep means produce biological effects due to the direct heating of the tissues located in greater depth. This group includes: shortwave or radio frequency, microwave and ultrasound, and according to the heat transfer mechanism, thermotherapy may be by conduction and heat convection, and by conversion of other forms of energy in the form of heat.

In this sense, different types and models of apparatuses to apply these heat treatments on different parts of the body are known in the market. Such apparatuses typically consist of an energy equipment and one or more means for transmission and application of such energy, e.g., electrodes or resistances, incorporated on an accessory element, which is the part that is positioned over the area or areas of the body to be treated, being connected to the apparatus via the corresponding cable.

Thus, in the case of treatments based on radio frequency, they are based on application, over the affected areas of the patient's body, of small electrical currents, at frequencies and powers determined with specific waveforms, through electrodes incorporated into aforementioned accessory elements for application.

On the other hand, the electrodes may be of two types:
- Monopolar, where the conducted radiofrequency is applied by means of two electrodes: one of return with fixed position, incorporated into a passive plate that is located under the area to be treated, and other active located on the other side of the area to be treated, and which may have a fixed position, incorporated into an active, or mobile, plate, incorporated into an element that moves.
- Bipolar, including the active and passive part of the radio frequency circuit in the same element. In turn, this bipolar electrode may be of fixed (bipolar plate), or mobile position.

In any case, whether they are radio frequency devices or another type of energy generator for thermotherapy treatments, depending on the parts of the body to which the application of treatment is intended, the element that includes the electrodes, resistances or means of energy transmission generated by the equipment to which are coupled, has one configuration or other, since it must be adapted to the intended area to allow such application in the best possible way.

As a reference to the current state of the art, it should be noted that, although there are known different types of accessories for the application of these treatments, with designs and structures specific for its use in different parts of the body, since, especially the contact surface, must be adapted to the morphology of the area, it is unaware of the existence of any other specifically applicable to intra-oral therapies or having the technical, structural and constitutive features of the one proposed herein, as claimed.

So, there are known, for example, flexible pads that are adapted to the surface of the skin in different areas of the body, such as that described in the document no. U9901071, the holder of which is the applicant itself, and it is related to a pad for the application of heat to the body by radio frequency currents. There are also known facial masks with specific structure for the application of facial treatments, such as that described in the document no. U9901344, also from the same applicant, where it is described a face mask for applying heat by radiofrequency. More specific treatment accessories are also known, such as the intracavitary element described in the document P201231383, applicable for therapies of gynaecology and urology, however, at least by the applicant, it is unknown the existence of any intra-oral element, i.e., intended to the application of thermal or athermal energy delivery treatments in different areas inside the mouth, the particular morphology of which requires a specific accessory adaptable to it.

The aim of the present invention is therefore the development of such element to cover its lack in the market and allow the specific application of such treatments inside the patients' mouth, thus allowing its use as additional support and/or as a therapeutic treatment specific in multiple types of oral conditions and or preferably being intended for its use by faculty dentists, for example, to avoid rejections in dental implantology and promote osseointegration of implants in bone, or prepare and stimulate the tissues in sessions both before and after an intervention for dental extraction or implant, favouring the regeneration of tissue, etc.

### EXPLANATION OF THE INVENTION

Thus, the intra-oral element for application of energy delivery treatments in jawbone, gums or other tissues inside the mouth proposed by the present invention is configured as a novelty within its field of application since, the characterizing details that distinguish it being suitably set down in the end claims accompanying the present specification.

So, the intra-oral element proposed by the invention, as already noted above, is a device applicable to be used with an energy equipment, as an accessory element to incorporate the means of transmission and application of energy generated by such equipment on tissues inside the mouth, having a structural configuration specifically designed for such use in order to apply heat-based treatments, with thermal or athermal effect more or less deep depending on different options of embodiment and as appropriate in each case, intended for its molecular stimulation, serving as a instrument in the treatment of oral conditions and/or as an aid, for example, in processes of dental implantology.

To do this, and more specifically, the intra-oral element is configured from a main body that is attached, at one end, to the cable that connects it to the energy equipment and at the opposite end to a shaft with a small head in which incorporates the means of application of such energy on the tissues to be treated, said main body being formed by an elongated housing in the form of handle, for easier handling, and the head being provided with an adapter, formed by a conductive medium with flexible structure, to ensure its adaptation and appropriate adjustment to the irregular morphology of the inside of the mouth.

In a preferred embodiment of the invention, the means for application of the energy on tissues to be treated consist of one or more electrodes, and the equipment to which are connected is a radio frequency electromagnetic wave equipment, conveniently provided with controls to adjust the frequencies and powers of the generated currents to produce specific waveforms to provide thermal or athermic effects in more or less deep areas of the tissues, while, optionally, such means may also consist of in simple electric resistances or other means that apply superficial heat, or even a combination of both.

In any case, the intra-oral element incorporates at least a temperature sensor since preferably the generating equipment has means to allow the quantitative measurement of the energy delivered, the incorporation of such means in the main body housing itself of the intra-oral element being an option of embodiment, comprising, in addition to the aforementioned sensor and corresponding electronics, a small screen, for example a monochrome liquid crystal display, through which displays the temperature and/or the power of energy delivered.

Also the intra-oral element of invention also contemplates the possibility of incorporating the control buttons for adjustment of such power, as well as, optionally, an emergency stop button that allows the immediate disconnection of energy delivery if necessary.

Still on the subject of the particularities of the intra-oral element proposed, it should be noted that, the means for application of energy incorporated in the head, that in an option of preferred embodiment are one or two electrodes, which cannot be ruled out that they may be of monopolar or bipolar type, are housed in an adapter formed by a conductive medium with flexible structure, which, preferably, consists of a gel pack.

In addition the shaft, at distal end of which is incorporated said head, is preferably a part removable and independent of the main body, so that it is an interchangeable part to allow its replacement in case of damage and to be able to attach to the intra-oral element different types and sizes of shaft and head, as a function of different needs, as well as for easy cleaning, while, optionally, the use of the intra-oral element may be carried out through the use of a disposable protective cover, to avoid the need for a continuous cleaning between patient and patient, thus ensuring a better hygiene.

On the other hand, the shaft, regardless of whether it is removable or not, also may be, optionally, of soft material, at least externally, in order to avoid damage to the patient and/or deterioration of the shaft itself by rubbings with hard parts, ensuring that any eventual contact is soft and, therefore at the same time avoiding mandibular stress usually caused by the introduction of hard instruments into the mouth.

Finally, the head and the shaft are preferably arranged at an angle for easy reach of the most inaccessible areas inside the mouth, as well as having the shaft itself with a curved configuration for said purpose.

The described intra-oral element for application of energy delivery treatments in jawbone, gums or other tissues inside the mouth thus represents an innovation of structural and constitutive characteristics unknown so far to this end, reasons which in combination with its practical utility, provide it with enough basis to obtain the exclusivity privilege which is applied for.

### DESCRIPTION OF THE DRAWINGS

In order to complement the description that is being carried out and with the object to. help to a better understanding of the invention, a set of drawings is accompanied to the present specification as an integral part thereof, in which, with an illustrative and non-limiting character, the following has been represented:

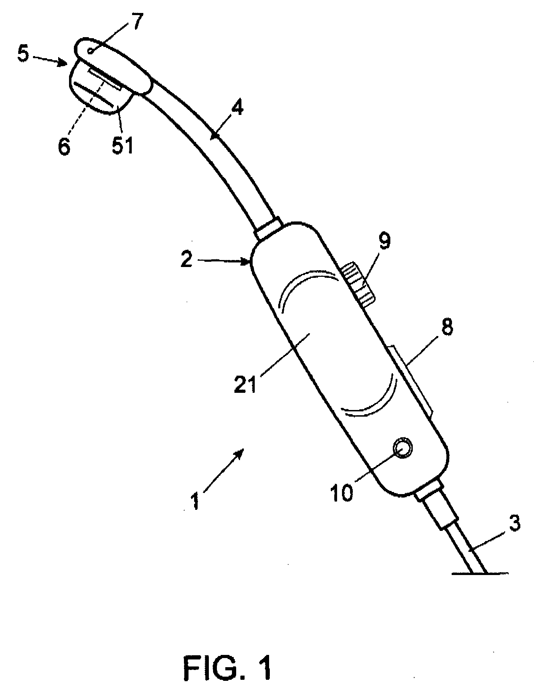

FIG. 1.- It shows a schematic side elevation view of an exemplary embodiment of the intra-oral element for application of energy delivery treatments in jawbone, gums or other tissues inside the mouth, object of the invention, being appreciated in it the main parts and elements that it comprises, as well as its configuration and arrangement.

### PREFERRED EMBODIMENT OF THE INVENTION

In light of the previously mentioned figures, and according to the numeration adopted, an example of a preferred and non-limiting embodiment of the intra-oral element can be observed therein, which comprises the parts and elements which are indicated and described in more detail below.

Thus, as shown in said figures, the intra-oral element (1) concerned is configured from a main body (2) attached at one end to a cable (3) that connects it to the energy equipment (not shown) and at the opposite end to a shaft (4) with a head (5) at its distal end where it incorporates the energy application means (6), formed by one or more electrodes, the equipment being a radiofrequency electromagnetic wave generator, electric resistances or a combination of both.

Furthermore, the intra-oral element (1) includes at least a temperature sensor (7) and, optionally, means to allow the quantitative measurement of the energy delivered, comprising, in addition to said sensor (7) and the necessary electronics incorporated into the main body (2), also a screen (8) displaying the temperature and/or the power of energy delivered.

Also, the intra-oral element (1) of the preferred embodiment includes control buttons (9) for power adjustment and, optionally, an emergency stop button (10), the operation of which determines the disconnection of energy delivery.

The main body (2) consists of a handle-shaped housing, preferably provided with lateral recesses (21) to facilitate its grip.

The shaft (4) is an elongated part, optionally removable from the main body, and interchangeable to allow its change or replacement by different types and sizes of shaft (4) and head (5), as a function of different needs, and for cleaning, being possible the use of a disposable protective cover, to ensure a better hygiene.

The shaft (4), regardless of whether it is removable or not, optionally, is also made of soft material, at least externally.

On the other hand, the head (5) is provided with an adapter (51), formed by a conductive medium with flexible structure covering the energy application means (6), formed by electrodes, electric resistances, or both, consisting of preferably a gel pack.

Finally, the head (5) and the shaft (4) are arranged at an angle or, as shown the example in FIG. 1, have a curved configuration that allows and easy reach of all areas inside the mouth, especially those most inaccessible.

Having sufficiently described the nature of the present invention, as well as the way of putting it into practice, it is not considered necessary to further extend its description for any person skilled in the art to understand its scope and the advantages derived therefrom, stating that, within its essence, it can be put into practice in other embodiments which differ only in detail from the one indicated by way of example, and which are also covered by the protection which is sought provided that its fundamental principle is not altered, changed or modified.

## Claims

1. Intra-oral element for application of energy delivery treatments in jawbone, gums or other tissues inside the mouth, in particular physiotherapy treatments, with thermal or athermal effect, based on radio frequency currents or other energies generated by a equipment to which is connected, **characterized in that** it is configured from a main body (2) attached at one end to a cable (3) that connects it to the energy equipment and at the opposite end to a shaft (4) with a head (5) at its distal end where it incorporates the energy application means (6).

2. Intra-oral element for application of energy delivery treatments in jawbone, gums or other tissues inside the mouth, according to claim 1, **characterized in that** the energy application means (6) consist of one or more electrodes, the equipment being a radiofrequency electromagnetic wave generator.

3. Intra-oral element for application of energy delivery treatments in jawbone, gums or other tissues inside the mouth, according to claim 1, **characterized in that** the energy application means (6) consist of electric resistances.

4. Intra-oral element for application of energy delivery treatments in jawbone, gums or other tissues inside the mouth, according to claim 1, **characterized in that** the energy application means (6) consist of one or more electrodes combined with electric resistances.

5. Intra-oral element for application of energy delivery treatments in jawbone, gums or other tissues inside the mouth, according to any of claims 1-4, **characterized in that** it includes at least one temperature sensor (7).

6. Intra-oral element for application of energy delivery treatments in jawbone, gums or other tissues inside the mouth, according to any of claims 1-5, **characterized in that** it incorporates means for quantitative measurement of the energy delivered, comprising a sensor (7) and the necessary electronics incorporated into the main body (2) as well as a screen (8) displaying the temperature and/or the power of energy delivered.

7. Intra-oral element for application of energy delivery treatments in jawbone, gums or other tissues inside the mouth, according to any of claims 1-6, **characterized in that** it includes control buttons (9).

8. Intra-oral element for application of energy delivery treatments in jawbone, gums or other tissues inside the mouth, according to any of claims 1-7, **characterized in that** it includes an emergency stop button (10) the operation of which determines the disconnection of energy delivery.

9. Intra-oral element for application of energy delivery treatments in jawbone, gums or other tissues inside the mouth, according to any of claims 1-8, **characterized in that** the main body (2) consist of a handle-shaped housing.

10. Intra-oral element for application of energy delivery treatments in jawbone, gums or other tissues inside the mouth, according to any of claims 1-9, **characterized in that** the shaft (4) is a piece removable from the main body and interchangeable to allow its replacement or change.

11. Intra-oral element for application of energy delivery treatments in jawbone, gums or other tissues inside the mouth, according to any of claims 1-10, **characterized in that** the shaft (4) at least externally is made of soft material.

12. Intra-oral element for application of energy delivery treatments in jawbone, gums or other tissues inside the mouth, according to any of claims 1-5, **characterized in that** the head (5) is provided with an adapter (51), formed by a conductive medium with flexible structure covering the energy application means (6).

13. Intra-oral element for application of energy delivery treatments in jawbone, gums or other tissues inside the mouth, according to claim 12, **characterized in that** the adapter (51) is a gel pack.

14. Intra-oral element for application of energy delivery treatments in jawbone, gums or other tissues inside the mouth, according to any of claims 1-13, **characterized in that** the head (5) and shaft (4) are arranged at an angle that allows an easy reach of all areas inside the mouth.

15. Intra-oral element for application of energy delivery treatments in jawbone, gums or other tissues inside the mouth, according to any of claims 1-13, **characterized in that** the head (5) and shaft (4) have a curved configuration that allows and easy reach of all areas inside the mouth.
